# EUROPÄISCHE PATENTSCHRIFT

(11) **EP 1 210 029 B1**
(45) Veröffentlichungstag und Bekanntmachung des Hinweises auf die Patenterteilung: **17.05.2006**
(21) Anmeldenummer: 00953177.3
(22) Anmeldetag: 18.08.2000
(51) Int. Cl.: A61C 13/15

(54) **BESTRAHLUNGSGERÄT**
IRRADIATION UNIT
APPAREIL D'IRRADIATION

(30) Priorität: 10.09.1999 DE 19943393
(43) Veröffentlichungstag der Anmeldung: 05.06.2002
(73) Patentinhaber: 3M ESPE AG, 82229 Seefeld (DE)
(72) Erfinder: HARTUNG, Martin, D-81541 München (DE)
(86) Internationale Anmeldenummer: PCT/EP2000/008058
(87) Internationale Veröffentlichungsnummer: WO 2001/019280

(56) Entgegenhaltungen:
- WO-A-99/16136
- WO-A-99/35995
- US-A- 5 549 660

## Beschreibung

Die Erfindung betrifft ein Bestrahlungsgerät mit hoher Lichtleistung, umfassend eine lichtemittierende Einheit, vorzugsweise LEDs, und eine lichtleitende Einheit mit einem lichtaufnehmenden Bereich, wie einen faseroptischen Lichtleiter, das vorzugsweise im medizinischen Bereich Anwendung findet.
Aus der JP-A-.9-10238 ist ein dentales Bestrahlungsgerät bekannt, bei dem ein Array aus Leuchtdioden an der Kalottenfläche eines kugelsektorförmigen Lichtleitkörpers aus Quarz oder Kunststoff angeordnet ist, dessen Spitze in einen Lichtleiterstab übergeht. Die Strahlen der Leuchtdioden werden von dem Lichtleitkörper durch Reflexion an der konischen Wandung gebündelt und in den Lichtleiterstab eingeleitet.
Die WO-97/36552 offenbart ein weiteres dentales Bestrahlungsgerät, bei dem ein planares Array aus Leuchtdioden mit parallelen optischen Achsen der gekrümmten Eingangsfläche eines wiederum konischen Lichtleitkörpers gegenübersteht. Dieser Kondensor ist ausgangsseitig an einen Lichtleiter gekoppelt und gegebenenfalls mit einer optische transparenten Flüssigkeit gefüllt.
Abgesehen davon, dass derartige konische Lichtleitkörper, wie sie in oben genannten Schriften, oder auch in der WO 99/16136 (Fig. 4) beschrieben wurden, in der Herstellung aufwendig sind und das Gewicht des Gerätes erhöhen, verursachen sie auch erhebliche Strahlungsverluste. Diese resultieren daraus, dass bei jeder Reflexion eines Lichtstrahls an der konusförmigen Wandung des Lichtleitkörpers der Strahl um das doppelte des Konuswinkels von der optischen Achse abgelenkt wird. Dies führt schon nach wenigen Reflexionen dazu, dass der Winkel für Totalreflexion im Lichtleiter überschritten wird und die Strahlen aus dem Lichtleiter austreten oder, im Falle einer Verspiegelung des Lichtleiters, die Strahlen sogar ihre Richtung umdrehen und somit nicht zur Lichtaustrittsöffnung sondern zu der Lichteintrittsöffnung zurück geleitet werden. Derartige Anordnungen funktionieren daher nur für den Anteil des von den LEDs eingestrahlten Lichtes, dessen Strahlen nur in einem sehr engen Winkelbereich gegenüber der optischen Achse verkippt sind. Somit kann der überwiegende Teil des von den LEDs abgestrahlten Lichts nicht zur Beleuchtung der Behandlungsfläche genutzt werden, da die Leuchtkegel, mit denen LEDs Licht abstahlen üblicherweise deutlich größere Öffnungswinkel aufweisen.
Aus der WO-99/16136 (Fig. 6) ist außerdem ein Gerät mit einem mehrfachkonischen Lichtleitkörper bekannt, bei dem mehrere ringförmige Lichteintrittsflächen einer kreisförmigen Lichteintrittsfläche vorangestellt sind. Dabei wird mit dem mehrfachkonischen Lichtleitkörper das Licht von der ersten kreisförmigen Lichteintrittsfläche in den Zentralbereich zwischen der ersten ringförmigen Lichteintrittsfläche gelenkt. Zusammen mit dem Licht der LEDs, die ringförmig angeordnet diese Lichteintrittsöffnung bestrahlen, wird es nunmehr in das Zentrum einer weiteren Kombination aus LED-Ring und ringförmiger Eintrittsfläche geleitet. Das so gesammelte Licht wird nun durch den im weiteren Verlauf nochmals konischen Lichtleiter zu einer Austrittsöffnung geführt.
Auch bei dieser Anordnung gelangt aus den oben genannten Gründen nur ein geringer Anteil der von den LED emittierten Strahlen zu der Lichtaustrittsöffnung und damit zu dem zu bestrahlenden Ort. Durch die Hintereinanderschaltung mehrerer konischer Lichtleitkörper ist die Effizienz der von der Austrittsöffnung am weitest entfernt gelegenen Bereiche sogar gegenüber einem einfachkonischen Kondensor nochmals reduziert. Zudem ist die Fertigung des mehrfachkonischen Lichtleiters nochmals aufwendiger und teurer.
Es sind auch andere Ausführungen von auf LED basierten Bestrahlungsgeräten, die ohne einen konischen Lichtleitkörper und die damit verbundenen Nachteile auskommen, bekannt. So werden in der JP 08-141001 (Fig. 1) und WO 99/35995 (Fig. 4) optische Sammellinsen zur Bündelung der von einem LED Array emittierten Strahlen und Fokussierung dieser auf die Lichteintrittsfläche eines Lichtleiters vorgeschlagen. Dabei wird die Gesamtheit der von den einzelnen LED's in Richtung der Sammellinse emittierten Strahlen abgelenkt. Die Ablenkung in die gewünschte Richtung und Fokussierung der Strahlen gelingt allerdings wiederum nur für den Anteil der Strahlen, die im wesentlichen parallel auf die Sammellinse auftreffen, oder, abhängig von der Größe der Lichteintrittsfläche, geringfügig von dieser Richtung abweichen. Ein erheblicher Anteil der Strahlen kann von der Sammellinse nicht auf die Lichteintrittsöffnung geleitet werden und ist somit für die Bestrahlung des Behandlungsortes verloren.
Bei der in Fig. 1 der WO 99/35995 gezeigten Anordnung werden 9 LEDs individuell in Richtung eines Lichtleiters ausgerichtet, wobei lediglich eine teilweise Durchhärtung einer lichthärtenden Probe beobachtet werden konnte. Dies ist auf unzureichende Lichtleistung in Folge nicht optimaler Lichteinkopplung sowie der geringen Anzahl der mit der beschriebenen Anordnung verwendbaren LEDs zurückzuführen.
Bei einem weiteren Gerät, dass in der WO 00/13608 beschrieben ist, und auf einer ähnlichen Anordnung der LEDs zu der Lichteintrittsöffnung eines Lichtleiters basiert, wird die Lichtleistung dadurch erhöht, dass zum einen ein konischer Lichtleiter verwendet, zum anderen die Dioden mit einem vielfachen des nominalen Arbeitsstromes beaufschlagt werden. Der konische Lichtleiter bringt die schon weiter oben beschriebenen Probleme mit sich. Nachteilig an den hohen Betriebsströmen ist, dass überproportional viel. Verlustwärme an den LEDs entsteht, wodurch das Gerät nach kurzer Zeit warm wird und längere Zeit bis zur Abkühlung nicht verwendet werden kann. Zudem leidet die Lebensdauer von LEDs stark unter den hohen Betriebsströmen. Dies äußert sich im Laufe der Zeit in einem kontinuierlichen Abfall der Lichtleistung.
Auch die in der WO 99/35995 oder der EP-A-0 879 582 vorgeschlagenen Anordnungen für LEDs in Form von einzelnen Halbleiterchips auf einem gemeinsamen Substrat sind nicht unproblematisch, da sich die einzelnen Elemente gegenseitig aufheizen, wodurch wiederum der Lichtintensität und/oder der Lebensdauer Grenzen gesetzt sind. Zudem ist die Herstellung derartiger Anordnungen wesentlich aufwendiger und teurer, da nicht auf Standardbauteile, die auch mechanisch gut zu handhaben sind, zurückgegriffen werden kann.

Obigen Geräten ist somit gemeinsam, dass die erreichbaren Lichtleistungen durch die dort beschriebenen Anordnungen der LEDs limitiert sind.
Folglich ist es eine Aufgabe der vorliegenden Erfindung, ein Bestrahlungsgerät mit verbesserter Lichtleistung bereitzustellen, womit z.B. eine schnellere und sicherere Durchhärtung von lichthärtenden dentalen Füllungsmaterialien erreicht werden kann.
Diese Aufgabe wird durch ein Bestrahlungsgerät gelöst, wie es in den Ansprüchen beschrieben ist.
Unter lichtemittierender Einheit im Sinne der Erfindung ist jede Form von Strahlungsquelle zu verstehen, die Licht erzeugt, vorzugsweise mit einer Wellenlänge im Bereich von 400 bis 500 nm. Vorzugsweise erfolgt diese Emission gerichtet. Die lichtemittierende Einheit umfasst üblicherweise eine Mehrzahl von lichtemittierenden Elementen.
Insbesondere fallen hierunter lichtemittierende Dioden (LED). Möglich ist aber auch der Einsatz von Laserdioden, wie sie in der EP-A-0 755 662 beschrieben werden.
Eine lichtaufnehmende Einheit im Sinne der Erfindung ist jede Vorrichtung, die fähig ist, Licht, das von der lichtemittierenden Einheit bzw. den lichtemittierenden Elementen erzeugt wird, aufzunehmen und gerichtet wieder abzugeben.
Üblicherweise handelt es sich hierbei um sogenannte Lichtleiterstäbe, die aus einer Vielzahl von gebündelten Glasfasern bestehen. Diese Lichtleiterstäbe sind über eine Kupplung oder ein Gewinde reversibel mit der lichtemittierenden Einheit bzw. dem diese enthaltenden Gehäuse verbindbar.
Umfasst sind aber auch herkömmliche Filterscheiben, Streulichtscheiben oder Lichtwellenkonverter, wie sie in der Anmeldung DE 100 06 286.5 beschrieben werden.
Die Begriffe "enthalten" und "umfassen" im Sinne der Erfindung leiten eine nichtabschließende Aufzählung von Merkmalen ein.
Die Erfindung beruht auf der überraschenden Erkenntnis, dass sich das Licht eines Leuchtdioden-Arrays mit gutem Wirkungsgrad auch ohne eine reflektorisch arbeitende Sammeloptik oder Sammellinsen auf die Lichteintrittsöffnung eines Lichtleiters bündeln und in diesen einkoppeln lässt.
Wesentlich für die Erzielung einer hohen Lichtleistung ist dabei die Erkenntnis, dass ein enger Zusammenhang zwischen der erzielbaren Lichtleistung und dem Abstrahlwinkel der lichtemittierenden Elemente, deren Abstand von der Lichteintrittsfläche sowie ihrer Ausrichtung zueinander sowie zu dem Lichtleiter besteht. Diese Abhängigkeit besteht darin, dass es besonders günstig ist, wenn die Lichtkegel der lichtemittierenden Elemente jeweils möglichst vollständig die Lichteintrittsfläche des Lichtleiters ausleuchten, d.h. die durch die Strahlenkegel ausgeleuchteten Flächen entsprechen im wesentlichen der Fläche der Eintrittsöffnung. Da der Querschnitt der Eintrittsöffnung fest vorgegeben ist, ergibt sich aus obiger Forderung über den Öffnungswinkel der Abstand der lichtemittierenden Elemente zur Eintrittsfläche. Würde dieser Abstand vergrößert, würde ein Teil des Lichtkegels an dem Lichtleiter vorbeileuchten und damit nicht nutzbar sein. Bei einem kleineren Abstand zum Lichtleiter, würde die Anzahl der lichtemittierenden Elemente, die auf dem Array Platz finden, reduziert werden.
Wie in den Ausführungsbeispielen beschrieben, ist es bei Berücksichtigung dieser Abhängigkeiten möglich, Anordnungen zu finden, bei denen der überwiegende Teil der von den einzelnen lichtemittierenden Elemente in Lichtkegeln emittierten Strahlung in einen Lichtleiter eingekoppelt und somit zu einem Behandlungsort geleitet wird und gleichzeitig eine möglichst große Anzahl von lichtemittierenden Elementen in dem Array integriert werden kann. Beides trägt zu einer möglichst hohen Lichtleistung des Bestrahlungsgerätes bei.
Geringe Unterschiede im Grad der Ausleuchtung, wie sie dadurch zwangsläufig zustande kommen, dass die eher im Zentrum angeordneten lichtemittierende Elemente ein näherungsweise kreisförmiges Beleuchtungsmuster, und die weiter am Rand angeordneten lichtemittierenden Elemente hingegen ein eher elliptisches Muster erzeugen, sind unvermeidlich und ohne größere praktische Relevanz. Wichtiger ist vielmehr, dass der Abstand der zentralen lichtemittierenden Elemente in der in den Abbildungen gezeigten Anordnungen derart bemessen ist, dass auch die elliptischen Leuchtflecke der am Rand gelegenen Elemente noch vollständig gesammelt werden können und nicht an der Eintrittsfläche vorbeileuchten.
Wesentlich ist dabei auch, dass die Strahlen unmittelbar auf die Lichteintrittsfläche gestrahlt werden, d.h. nicht in ihrer Richtung durch z.B. Sammellinsen oder reflektorisch arbeitende Kondensor-Optiken abgelenkt werden, da eine solche Ablenkung immer nur für einen kleinen Anteil der Strahlen günstig in Bezug auf ihre Einkoppelbarkeit in einen Lichtleiter ist. Strahlen mit schon geringfügig anderer Richtung können aber nach der Ablenkung nicht mehr genutzt werden, weil sie an der Eintrittsöffnung vorbeileuchten oder der maximale Winkel, unter dem Strahlen noch in einen Lichtleiter eingekoppelt werden können, dem sogenannten Akzeptanzwinkel, überschritten ist.
Überraschenderweise lässt sich durch die erfindungsgemäße Anordnung der lichtemittierenden Elemente in mindestens zwei von einander getrennten Ebenen die mit dem Bestrahlungsgerät erzielbare Lichtleistung sogar noch erhöhen, ohne dass zwischen den Ebenen lichtleitende oder lichtfokussierende Elemente vorhanden zu sein brauchen.
Vorteilhaft ist auch, dass sich durch diese Anordnung das Bestrahlungsgerät verhältnismäßig kompakt und handlich gestalten lässt, ohne dass die zur Verfügung stehende Lichtleistung darunter leidet.
Auch bei der Anordnung in mindestens zwei Ebenen ist es besonders vorteilhaft, wenn die Lichtkegel die Eintrittsfläche im wesentlichen vollständig ausleuchten. Da die Ebenen in unterschiedlichem Abstand zu der Eintrittsöffnung angeordnet sind, sind unterschiedliche Abstrahlwinkel der lichtemittierenden Elemente in den einzelnen Ebenen vorteilhaft.
Lichtemittierende Elemente, die in der ersten, der Eintrittsöffnung am nächsten gelegenen Ebene angeordnet sind, weisen vorzugsweise einen größeren Abstrahlwinkel auf als die übrigen lichtemittierenden Elemente der nachgeordneten Ebene(n). Dies ist insbesondere auch deshalb vorteilhaft, da lichtemittierende Elemente mit einem relativ großen Abstrahlwinkel von z.B. +/- 30° (z.B. Nichia NSPB 310A) ca. 70% ihrer gesamten Strahlung in den nach vorne gerichteten und nutzbaren Lichtkegel emittieren. Für lichtemittierende Elemente mit engeren Abstrahlwinkeln von z.B. +/- 15° (z.B. Nichia NSPB 300A) beträgt dieser Anteil nur noch etwa 50%. Der Rest der Strahlung wird seitlich und nicht nutzbar abgestrahlt. Lichtemittierende Elemente, die weiter vom Lichtleiter entfernt sind, müssen ihr Licht aber in engere Lichtkegel emittieren, damit diese vollständig auf der Eintrittsöffnung auftreffen und somit gesammelt werden können.
Vorteilhaft ist auch, wenn der Abstand der einzelnen Ebenen zum Lichtleiter und untereinander so klein wie möglich ist. Ein Abstand zwischen den einzelnen Ebenen, der im wesentlichen der Baulänge der verwendeten lichtemittierenden Elemente entspricht, hat sich als günstig erwiesen.
Gegebenenfalls wird die lichtemittierende Einheit mit einer transparenten Scheibe abgedeckt sein, um die lichtemittierenden Elemente bei abgenommenen Lichtleiter vor Feuchtigkeit und Verschmutzung zu schützen. Als besonders vorteilhaft hat es sich erwiesen, diese Abdeckscheibe in Form einer Prismenscheibe auszuführen, da dadurch die Lichtleistung nochmals gesteigert werden kann. Vorraussetzung dafür ist allerdings, dass die Prismenscheibe die unmittelbare Bestrahlung der Eintrittsfläche der Lichtaufnehmenden Einheit nicht stört, d.h. die Richtung der auf diese gerichteten Lichtstrahlen nicht beeinflusst, aber auch nicht abschwächt. Letzteres kann leicht durch eine optische Vergütung erreicht werden.
Eine Prismenscheibe im Sinne der Erfindung ist eine transparente Vorrichtung in der Form eines verhältnismäßig flachen Kegelstumpfes mit einer ebenen Ober- und im Durchmesser kleineren Unterseite, wobei der Durchmesser der Unterseite im wesentlichen dem Durchmesser der lichtaufnehmenden Einheit entspricht.
Die Prismenscheibe verfügt über zwei sich optisch unterschiedlich verhaltende Bereiche, einem planen zylinderförmigen Bereich und einem rotationssymmetrischen prismenförmigen Randbereich. Die Prismenscheibe weist keinen einheitlichen Brennpunkt auf und funktioniert überwiegend nach refraktorischen und nicht nach reflektorischen Gesichtspunkten.
Die zylinderförmige Einheit lenkt senkrecht auftreffende Lichtstrahlen nicht und schräg auftreffende Lichtstrahlen gemäß dem Snellius'schen Brechungsgesetz ab, wobei zwischen ein- und austretendem Strahl lediglich eine geringfügige Parallel-Verschiebung resultiert. Die Richtung der Strahlen bleibt jedoch im wesentlichen unverändert.
Die Funktion des prismenförmigen Randbereichs besteht darin, Licht, das von den der Eintrittsöffnung am nächsten liegenden lichtemittierenden Elementen trotz optimierter Anordnung seitlich an der Eintrittsöffnung vorbeigestrahlt werden würde, gerichtet auf die Eintrittsöffnung umzulenken.
Die Eintrittsöffnung wird durch Anbringen einer solchen Prismenscheiben im Durchmesser optisch gleichsam vergrößert, ohne, dass im Zentralbereich eine nennenswerte Störung des Strahlenganges erfolgt.
Die Prismenscheibe kann sowohl mit der Unterseite als auch mit der Oberseite parallel zur lichtaufnehmenden Einheit orientiert werden.
Denkbar ist auch, zwei Prismenscheiben aufeinander zu legen, sodass ein diskusförmiges Gebilde entsteht.
Die Prismenscheibe kann aus Glas oder transparentem Kunststoff gefertigt sein. Das Material weist vorzugsweise einen Brechungsindex im Bereich von 1,4 bis 1,6 auf.
Vorzugsweise ist die Prismenscheibe in einem Abstand, der ihrer Stärke bzw. Höhe entspricht, parallel zur Eintrittsöffnung des lichtaufnehmenden Elements angeordnet. Der Raum zwischen Prismenscheibe und Eintrittsöffnung ist üblicherweise luftgefüllt.
Die lichtemittierenden Elemente werden vorzugsweise auf nur einer, üblicherweise flachen Platine montiert, um Fertigungskosten zu sparen. Durch eine unterschiedliche Länge der Anschlussdrähte für die lichtemittierenden Elemente lassen sich einzelne, voneinander getrennte Ebenen erzeugen.
Das Problem der Abfuhr der beim Betrieb des Bestrahlungsgerätes von den lichtemittierenden Elementen erzeugten Wärme lässt sich durch Verwendung einer doppelseitig kaschierten Platine lösen. Hierzu werden die die Verlustwärme erzeugenden Kathoden auf der Unterseite und die Anoden auf der Oberseite angebracht. Da sich auf der Platinenunterseite üblicherweise eine Wärmesenke, beispielsweise in Form eines Cu- oder Al-Körpers, befindet, ist die Wärmeübertragung verbessert.
Das Bestrahlungsgerät verfügt üblicherweise auch über eine elektronische Steuereinheit zur Steuerung von Spannung und Stromstärke für die lichtemittierenden Elemente, eine Speichereinheit für elektrische Energie, wie Batterien oder Akkumulatoren, vorzugsweise Lithiumionen-, NiMH- oder Ni/Cd-Akkumulatoren, eine Anzeigeeinheit und ein Gehäuse.
Das Gehäuse ist vorzugsweise derart gestaltet, dass es im wesentlichen eine spaltenfreie Oberfläche aufweist, in die die Anzeigeinheit integriert ist.

Bevorzugte Ausführungsbeispiele werden nachstehend anhand der Zeichnungen erläutert.
Figur 1 zeigt einen Längsschnitt durch das Bestrahlungsgerät.
Figur 2 zeigt eine vergrößerte Darstellung des vorderen Teils des Geräts nach Figur 1.
Figur 3 zeigt eine weitere Ausführungsform des Frontbereichs des Bestrahlungsgeräts.

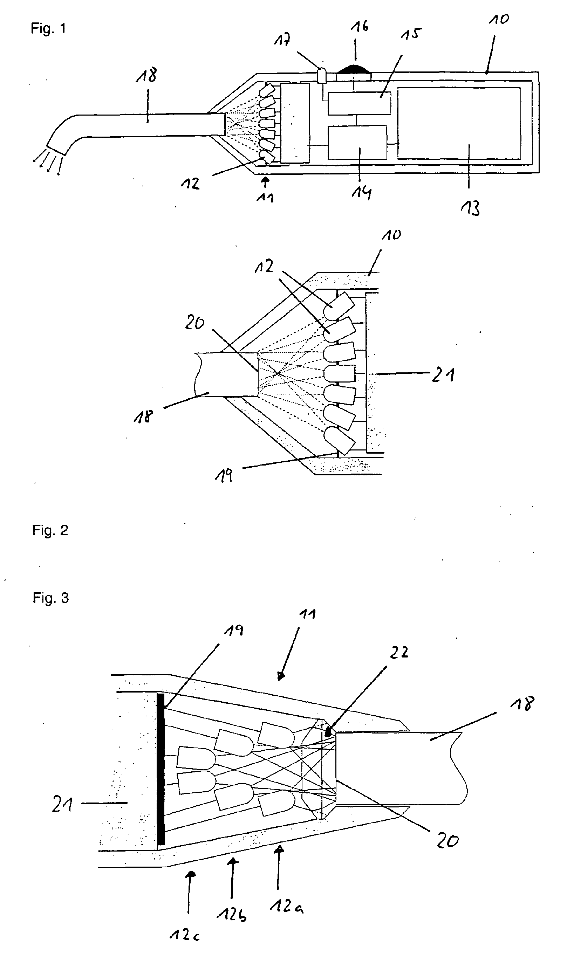

Das in Figur 1 gezeigte Handgerät zum Bestrahlen von dentalen Kunststoffen enthält im vorderen Bereich eines im wesentlichen zylindrischen Gehäuses (10) eine lichtemittierende Einheit (11) in Form eines Leuchtdioden-Arrays mit beispielsweise fünfzig einzelnen lichtemittierenden Elementen (12), wie Leuchtdioden, bei denen es sich auch um Laserdioden handeln kann.
Die lichtemittierenden Elemente (12) werden aus einer, im hinteren Teil des Gehäuses (10) angeordneten Batterie (13) über eine Treiberstufe (14) gespeist, die von einer Steuerschaltung (15) zeitgesteuert wird. Die Steuerschaltung (15) ist mit einem seitlich am Gehäuse (10) angeordneten Einschalttaster (16) und einer ebenfalls seitlich am Gehäuse (10) angeordneten Anzeigediode (17) verbunden. Aus dem vorderen konischen Ende des Gehäuses (10) ragt eine lichtaufnehmende Einheit (18) in Form eines an seinem vorderen Ende gekrümmten Lichtleiterstabs heraus.
Die lichtemittierenden Elemente (12) sind in dieser Ausführungsform in einer ebenen Halteplatte (19) derart angeordnet, dass ihre optischen Achsen einander in einem Fokusbereich (20) schneiden, der an dem innerhalb des Gehäuses (10) befindlichen, mit einer Antireflexionsschicht versehenen Eintrittsende der lichtaufnehmenden Einheit (18) liegt.
Der mit dem Abstand von der Mittelachse zunehmende Winkel, um den die optische Achse jedes lichtemittierenden Elements (12) gekippt ist, ist unter Berücksichtigung der Strahlkegelöffnung und des Abstands vom Lichtleiterstab (18) so gewählt, dass im wesentlichen das gesamte Strahlungsbündel der lichtemittierenden Elemente (12) auf die Eintrittsfläche des Lichtleiterstabes (18) fällt und diesen im wesentlichen vollständig ausleuchtet. Damit das, auf die Lichteintrittsfläche treffende Licht auch für die Bestrahlung des Behandlungsortes genutzt werden kann, muss es in den Lichtleiter eingekoppelt werden. Deshalb darf der Winkel, unter dem die Strahlen auf die Eintrittsfläche auftreffen nicht größer als der maximale Akzeptanzwinkel des Lichtleitstabes sein. Eine solche planare Anordnung der lichtemittierenden Elemente mit gekippten Optischeachse ist aus WO 99 35995 (Ausführung der Figur 1) bekannt. Dies begrenzt die Anzahl der lichtemittierenden Elemente, die in der gezeigten Anordnung sinnvollerweise integriert werden können. Wenn der Abstand der lichtemittierenden Elemente zu der Eintrittsfläche bei in diesem Falle möglichst engem Abstrahlwinkel der lichtemittierenden Elemente derart gewählt ist, dass der Lichtleiter möglichst vollständig ausgeleuchtet wird und auch möglichst kein Licht an diesem vorbeileuchtet, ist ein Optimum der Anzahl von Elementen gegeben. Abstrahlwinkel der lichtemittierenden Elemente im Bereich von etwa +/- 15° haben sich bei diesem Ausführungsbeispiel besonders bewährt.
Anstelle der oben beschriebenen planaren Anordnung können die lichtemittierenden Elemente auch an einer gekrümmten, insbesondere sphärischen, zum Fokusbereich hin konkaven Fläche angeordnet sein. Dies hat den Vorteil, dass dann die Abstände der lichtemittierenden Elemente zu dem Lichtleiter identisch sind, was bei einer ebenen Anordnung nicht exakt erreicht werden kann. Die Größen der Leuchtflecken, die durch die einzelnen Leuchtkegel auf der Lichteintrittsöffnung erzeugt werden stimmen dann noch besser mit dieser überein.
Jeweils eine der Zuleitungen der lichtemittierenden Elemente (12), vorzugsweise die Kathode, ist mit einem Körper (21) aus einem Werkstoff hoher Wärmeleitfähigkeit und -kapazität, vorzugsweise Kupfer und/oder Aluminium, thermisch verbunden, der als Wärmesenke für die Leuchtdioden (12) herangezogen wird.
Da die lichtemittierenden Elemente (12) in dieser Ausführungsform in einer gemeinsamen planaren oder gekrümmten Ebene liegen, hat der Wärmesenkekörper (21), der für optimale Wirkung möglichst nahe an den lichtemittierenden Elementen (12) angeordnet sein soll, in dieser Ausführungsform die Gestalt einer zu der Haltescheibe (19) parallel angeordneten planparallelen Scheibe. Dies ist unter dem Gesichtspunkt sowohl eines geringen Herstellungsaufwandes als auch einer gedrängten Bauform günstig.
Figur 3 zeigt eine weitere Ausführungsform des Frontbereichs des Bestrahlungsgeräts im Querschnitt mit einer lichtemittierenden Einheit (11), einer lichtaufnehmenden Einheit (18), einer Mehrzahl lichtemittierenden Elementen (12), die auf drei Ebenen angeordnet sind (12a, 12b, 12c), wobei der Öffnungswinkel der lichtemittierenden Elemente der ersten Ebene (12a), die der Eintrittsöffnung am nächsten angeordnet ist, größer ist, als der Öffnungswinkel der lichtemittierenden Elemente der zweiten (12b) und dritten Ebene (12c), die von der Eintrittsöffnung weiter entfernt angeordnet sind.
Die lichtemittierenden Elemente der ersten und zweiten Ebene sind auf einer Ringfläche angeordnet, während sich die lichtemittierenden Elemente der dritten Ebene auf einer Kreisfläche befinden. Angedeutet ist auch der je nach Ebene unterschiedliche Verkippungsgrad der lichtemittierenden Elemente zur Ringmitte hin. Damit ist sichergestellt, dass die Leuchtkegel der zweiten und dritten Ebene zum einen an den lichtemittierenden Elementen der vorgelagerten Ebenen vorbeileuchten können, zum anderen aber auf die Lichteintrittsöffnung auftreffen und diese vollständig ausleuchten.

In dem gezeigten Ausführungsbeispiel befindet sich zwischen lichtemittierender Einheit (11) und lichtaufnehmender Einheit (18) eine Prismenscheibe (22), die in ihrem Randbereich das von den lichtemittierenden Elementen der ersten Ebene (12a) seitlich an der Eintrittsöffnung der lichtaufnehmenden Einheit vorbei gestrahlte Licht zur Eintrittsöffnung hin brechen. Mit der Prismenscheibe, kann insbesondere auch der Durchmesser der ersten Ebene vergrößert werden. Damit findet zum einen eine größere Anzahl von lichtemittierenden Elementen auf diesem Ring Platz, zum anderen vergrößert sich die kreisförmige Öffnung im Zentrum dieser Ebene, wodurch auch in den nachgelagerten Ebenen eine größere Anzahl von lichtemittierenden Elementen untergebracht werden kann. Durch den planaren Zentralbereich, dessen Durchmesser dem Durchmesser der Lichteintrittsöffnung in etwa entspricht, ist sichergestellt, dass diese nach wie vor unmittelbar bestrahlt werden kann, d.h. die auf diese gerichtete Strahlen nicht in ihrer Richtung abgelenkt werden. Mit dem prismenförmigen Randbereich wird aber zusätzlich Strahlung auf den Lichtleiter gelenkt, die somit die Lichtleistung des Gerätes weiter steigert.
Die lichtemittierenden Elemente sind im gezeigten Ausführungsbeispiel alle auf einer Halteplatte (19) montiert, an die sich auf der Unterseite eine Wärmesenke (21) in Form eines Cu-Körpers befindet.
Das erfindungsgemäße Belichtungsgerät findet insbesondere im Medizinbereich, vorzugsweise im Dentalbereich Anwendung und kann einerseits zum Ausleuchten des Behandlungsortes oder zum Bestrahlen von durch Licht härtbaren Massen, insbesondere dentalen Füllungsmaterialien, wie Compositen, Compomeren oder Glasionomerzementen dienen.
Im Gebrauch wird das Austrittsende des Lichtleiterstabes auf die Behandlungsstelle, etwa eine auszuhärtende Zahnfüllung, gerichtet und der Einschalttaster gedrückt, wodurch die Leuchtdioden beaufschlagt werden und gleichzeitig die Anzeigeeinheit eingeschaltet wird. Nach einer vorgegebenen oder einstellbaren Zeitspanne schaltet die Steuerschaltung die Stromzufuhr zu den Leuchtdioden und der Anzeigeeinheit ab.

### Bezugszeichenliste:

- 10: Gehäuse
- 11: lichtemittierende Einheit/Array
- 12: lichtemittierende Elemente/Leuchtdioden
- 13: Batterie
- 14: Treiberstufe
- 15: Steuerschaltung
- 16: Einschalttaster
- 17: Anzeigeeinheit
- 18: lichtaufnehmende Einheit/Lichtleiterstab
- 19: Halteplatte
- 20: Fokusbereich
- 21: Wärmesenkenkörper
- 22: Prismenscheibe

## Patentansprüche

1. Bestrahlungsgerät, umfassend eine lichtemittierende Einheit (11) und eine lichtaufnehmende Einheit (18) mit einer Eintrittsöffnung, wobei die lichtemittierende Einheit (11) eine Mehrzahl von lichtemittierenden Elementen (12) umfasst, deren in Form eines Kegels gerichtet emittierte Lichtstrahlen auf die Eintrittsöffnung gerichtet sind und diese unmittelbar bestrahlen, wobei die Abstände der lichtemittierenden Elemente von der Eintrittsöffnung, die Öffnung der Strahlenkegel der lichtemittierenden Elemente, sowie die Neigungswinkel ihrer optischer Achsen derart gewählt sind, dass die durch die jeweiligen Strahlenkegel ausgeleuchteten Flächen, im wesentlichen der Fläche der Eintrittsöffnung entsprechen, **dadurch gekennzeichnet, dass** die lichtemittierenden Elemente (12) in mindestens zwei Ebenen parallel zur Eintrittsöffnung angeordnet sind, und die lichtemittierenden Elemente der der Eintrittsöffnung nächstliegenden Ebene (12a) auf einer Ringfläche und die lichtemittierenden Elemente der der Eintrittsöffnung am weitesten entfernt liegenden Ebene (12c) auf einer Kreisfläche angeordnet sind, wobei der von den Elementen der von der Eintrittsöffnung am weitesten entfernt liegenden Ebene emittierte Lichtkegel im wesentlichen vollständig durch die jeweils oberhalb angeordnete Ringöffnung der vorgelagerten Ebene hindurch auf die Eintrittsöffnung trifft, und wobei sich im Bereich zwischen den mindestens zwei Ebenen keine zusätzlichen lichtfokussierenden oder lichtleitenden Elemente befinden.

2. Bestrahlungsgerät nach Anspruch 1, wobei die lichtemittierenden Elemente (12) auf drei Ebenen angeordnet sind.

3. Bestrahlungsgerät nach einem der Ansprüche 1 oder 2, wobei der Durchmesser des Kreises der der Eintrittsöffnung nächstliegenden Ebene (12a), gemessen an der Spitze der lichtemittierenden Elemente im wesentlichen dem Durchmesser der Eintrittsöffnung entspricht.

4. Bestrahlungsgerät nach einem der Ansprüche 1 bis 3, wobei der Durchmesser des Rings der ringförmig angeordneten lichtemittierenden Elemente größer ist als der Durchmesser der Kreisfläche der von der Eintrittsöffnung am weitesten entfernt liegenden Ebene.

5. Bestrahlungsgerät nach einem der Ansprüche 1 bis 4, wobei der Abstand der einzelnen Ebenen im wesentlichen der Länge eines lichtemittierenden Elements entspricht.

6. Bestrahlungsgerät nach einem der Ansprüche 1 bis 5, wobei die Lichtkegel der lichtemittierenden Elemente der ersten und zweiten und der gegebenenfalls vorhandenen dritten Ebene unterschiedliche Öffnungswinkel aufweisen.

7. Bestrahlungsgerät nach einem der Ansprüche 1 bis 6, wobei der Öffnungswinkel der lichtemittierenden Elemente der der Eintrittsöffnung am nächsten liegenden Ebene (12a) größer ist als der Öffnungswinkel der lichtemittierenden Elemente der der Eintrittsöffnung entfernter liegenden anderen Ebenen (12b, 12c).

8. Bestrahlungsgerät nach einem der Ansprüche 1 bis 7, wobei die lichtemittierenden Elemente, die ringförmig angeordnet sind, um einen Winkel im Bereich von 10° bis 30° zum Ringzentrum hin verkippt sind.

9. Bestrahlungsgerät nach einem der vorstehenden Ansprüche, wobei die lichtemittierenden Elemente mit einem Gehäuse (10) thermisch verbunden sind.

10. Bestrahlungsgerät nach einem der vorstehenden Ansprüche, wobei die lichtemittierenden Elemente mit unterschiedlichen Neigungswinkeln in einer im wesentlichen planaren Halterung (19) angeordnet sind.

11. Bestrahlungsgerät nach Anspruch 10, wobei es sich bei der Halterung (19) um eine doppelseitig kaschierte Platine handelt, auf der die Anoden der lichtemittierenden Elemente auf der Oberseite und die Kathoden auf der Rückseite der Platine kontaktiert sind.

12. Bestrahlungsgerät nach einem der vorstehenden Ansprüche, wobei die lichtaufnehmende Einheit (18) gewählt ist aus einem starren Lichtleiterstab oder einem flexiblen Lichtleiter.

13. Bestrahlungsgerät nach einem der vorstehenden Ansprüche, wobei sich zwischen lichtemittierender Einheit (11) und lichtaufnehmender Einheit (12) eine Prismenscheibe (22) befindet.

14. Bestrahlungsgerät nach Anspruch 13, wobei die Prismenscheibe (22) die Form eines flachen Kegelstumpfes hat, dessen kleinerer Durchmesser im wesentlichen dem Durchmesser der Eintrittsöffnung entspricht.

15. Bestrahlungsgerät nach einem der Anspruch 13 oder 14, wobei die im Durchmesser kleinere unterseite der Prismenscheibe (22) der lichtemittierenden den Einheit zugewandt ist.

16. Bestrahlungsgerät nach einem der vorstehenden Ansprüche , wobei der Durchmesser der Eintrittsöffnung im Bereich von 8 bis 14 mm liegt und sich in der ersten Ebene 8 bis 15, in der zweiten Ebene 5 bis 12 und in der gegebenenfalls vorhandenen dritten Ebene 1 bis 7 lichtemittierende Elemente befinden.

## Claims

1. Irradiation unit comprising a light-emitting unit (11) and a light-absorbing unit (18) with an entrance orifice, the light-emitting unit (11) comprising a plurality of light-emitting elements (12) whose light beams, which are emitted directed in the shape of a cone, are directed onto the entrance orifice and directly irradiate the latter, the distances of the light-emitting elements from the entrance orifice, the aperture of the beam cones of the light-emitting elements and the angle of inclination of their optical axes being selected in such a way that the surfaces illuminated by the respective beam cones correspond substantially to the surface of the entrance orifice, **characterized in that** the light-emitting elements (12) are arranged in at least two planes parallel to the entrance orifice, and the light-emitting elements of the plane (12a) situated closest to the entrance orifice are arranged on an annular surface and the light-emitting elements of the plane (12c) situated furthest removed from the entrance orifice are arranged on a circular surface, the light cone emitted by the elements of the plane situated furthest removed from the entrance orifice impinging substantially completely on the entrance orifice through the annular opening, respectively arranged above, of the plane in front, and no additional light-focusing or light-guiding elements being located in the region between the at least two planes.

2. Irradiation unit according to Claim 1, in which the light-emitting elements (12) are arranged on three planes.

3. Irradiation unit according to one of Claims 1 or 2, in which the diameter of the circle of the plane (12a) situated closest to the entrance orifice, measured at the tip of the light-emitting elements, corresponds substantially to the diameter of the entrance orifice.

4. Irradiation unit according to one of Claims 1 to 3, in which the diameter of the ring of the annularly arranged light-emitting elements is greater than the diameter of the circular surface of the plane situated furthest removed from the entrance orifice.

5. Irradiation unit according to one of Claims 1 to 4, in which the distance of the individual planes corresponds substantially to the length of a light-emitting element.

6. Irradiation unit according to one of Claims 1 to 5, in which the light cones of the light-emitting elements of the first and second and the possibly present third plane have different aperture angles.

7. Irradiation unit according to one of Claims 1 to 6, in which the aperture angle of the light-emitting elements of the plane (12a) situated closest to the entrance orifice is greater than the aperture angle of the light elements of the other planes (12b, 12c) situated further removed from the entrance orifice.

8. Irradiation unit according to one of Claims 1 to 7, in which the light-emitting elements, which are arranged annularly, are tilted toward the centre of the ring by an angle in the range from 10° to 30°.

9. Irradiation unit according to one of the preceding claims, in which the light-emitting elements are thermally connected to a housing (10).

10. Irradiation unit according to one of the preceding claims, in which the light-emitting elements are arranged with different angles of inclination in a substantially planar holder (19).

11. Irradiation unit according to Claim 10, in which the holder (19) is a circuit board coated on both sides on which contact is made with the anodes of the light-emitting elements on the top side, and contact is made with the cathodes on the rear side of the circuit board.

12. Irradiation unit according to one of the preceding claims, in which the light-absorbing unit (18) is selected from a rigid optical fibre rod or a flexible optical conductor.

13. Irradiation unit according to one of the preceding claims, in which a prismatic disc (22) is located between the light-emitting unit (11) and the light-absorbing unit (12).

14. Irradiation unit according to Claim 13, in which the prismatic disc (22) has the shape of a flat conical frustum whose smaller diameter corresponds substantially to the diameter of the entrance orifice.

15. Irradiation unit according to one of Claims 13 or 14, in which the underside of the prismatic disc (22), which is smaller in diameter, faces the light-emitting unit.

16. Irradiation unit according to one of the preceding claims, in which the diameter of the entrance orifice is in the range from 8 to 14 mm, and 8 to 15 light-emitting elements are located in the first plane, 5 to 12 in the second plane, and 1 to 7 in the possibly present third plane 1.

## Revendications

1. Appareil d'irradiation comprenant une unité émettrice de lumière (11) et une unité réceptrice de lumière (18) comprenant une ouverture d'entrée, l'unité émettrice de lumière (11) comprenant une pluralité d'éléments émetteurs de lumière (12) dont les rayons lumineux émis sont dirigés sur l'ouverture d'entrée sous la forme d'un cône et irradient directement celle-ci, les écarts entre les éléments émetteurs de lumière et l'ouverture d'entrée, l'ouverture du cône des rayons des éléments émetteurs de lumière ainsi que l'angle d'inclinaison de leurs axes optiques étant choisis de telle sorte que les surfaces éclairées par le cône de rayons correspondant correspondent pour l'essentiel à la surface de l'ouverture d'entrée, **caractérisé en ce que** les éléments émetteurs de lumière (12) sont disposés sur au moins deux plans parallèlement à l'ouverture d'entrée et les éléments émetteurs de lumière du plan (12a) le plus proche de l'ouverture d'entrée étant disposés sur une surface annulaire et les éléments émetteurs de lumière du plan (12c) le plus éloigné de l'ouverture d'entrée étant disposés sur une surface circulaire, le cône de lumière émis par les éléments du plan le plus éloigné de l'ouverture d'entrée venant frapper pour l'essentiel entièrement l'ouverture d'entrée à travers l'ouverture annulaire du plan suivant disposée à chaque fois au-dessus et aucun élément de concentration de la lumière ou de guidage de la lumière ne se trouvant dans la zone entre les au moins deux plans.

2. Appareil d'irradiation selon la revendication 1, les éléments émetteurs de lumière (12) étant disposés sur trois plans.

3. Appareil d'irradiation selon l'une des revendications 1 ou 2, le diamètre du cercle du plan (12a) le plus proche de l'ouverture d'entrée, mesuré à la pointe des éléments émetteurs de lumière, correspondant pour l'essentiel au diamètre de l'ouverture d'entrée.

4. Appareil d'irradiation selon l'une des revendications 1 à 3, le diamètre de l'anneau des éléments émetteurs de lumière disposés en forme d'anneau étant supérieur au diamètre de la surface circulaire du plan le plus éloigné de l'ouverture d'entrée.

5. Appareil d'irradiation selon l'une des revendications 1 à 4, l'écart entre les plans individuels correspondant pour l'essentiel à la longueur d'un élément émetteur de lumière.

6. Appareil d'irradiation selon l'une des revendications 1 à 5, les cônes de lumière des éléments émetteurs de lumière du premier et du deuxième ainsi que du troisième plan éventuellement présent présentant des angles d'ouverture différents.

7. Appareil d'irradiation selon l'une des revendications 1 à 6, l'angle d'ouverture des éléments émetteurs de lumière du plan (12a) le plus proche de l'ouverture d'entrée étant supérieur à l'angle d'ouverture des éléments émetteurs de lumière des autres plans (12b, 12c) plus éloignés de l'ouverture d'entrée.

8. Appareil d'irradiation selon l'une des revendications 1 à 7, les éléments émetteurs de lumière qui sont disposés en forme d'anneau étant inclinés selon un angle compris entre 10° et 30° en direction du centre de l'anneau.

9. Appareil d'irradiation selon l'une des revendications précédentes, les éléments émetteurs de lumière étant reliés de manière thermique avec un boîtier (10).

10. Appareil d'irradiation selon l'une des revendications précédentes, les éléments émetteurs de lumière étant disposés selon des angles d'inclinaison différents dans un support (19) pour l'essentiel plan.

11. Appareil d'irradiation selon la revendication 10, le support (19) étant une platine stratifiée des deux côtés sur laquelle les anodes des éléments émetteurs de lumière sont mises en contact sur le dessus et les cathodes sur le dos de la platine.

12. Appareil d'irradiation selon l'une des revendications précédentes, l'unité réceptrice de lumière (18) étant choisie dans une barre en fibres optiques rigide ou une fibre optique souple.

13. Appareil d'irradiation selon l'une des revendications précédentes, un disque à prismes (22) se trouvant entre l'unité émettrice de lumière (11) et l'unité réceptrice de lumière (12).

14. Appareil d'irradiation selon la revendication 13, le disque à prismes (22) ayant la forme d'un cône tronqué plat dont le petit diamètre correspond pour l'essentiel au diamètre de l'ouverture d'entrée.

15. Appareil d'irradiation selon l'une des revendications 13 ou 14, le dessous du disque à prismes (22) dont le diamètre est plus petit faisant face à l'unité émettrice de lumière.

16. Appareil d'irradiation selon l'une des revendications précédentes, le diamètre de l'ouverture d'entrée étant compris entre 8 et 14 mm et le premier plan comprenant 8 à 15, le deuxième plan 5 à 12 et le troisième plan éventuellement présent 1 à 7 éléments émetteurs de lumière.
